(19) **Europäisches Patentamt**
**European Patent Office**
**Office européen des brevets**

(11) **EP 1 723 987 B1**

(12) **EUROPEAN PATENT SPECIFICATION**

(45) Date of publication and mention
of the grant of the patent:
**18.07.2012 Bulletin 2012/29**

(51) Int Cl.:
***A61N 5/06*** (2006.01)

(21) Application number: **05722143.4**

(86) International application number:
**PCT/RU2005/000038**

(22) Date of filing: **04.02.2005**

(87) International publication number:
**WO 2005/079918 (01.09.2005 Gazette 2005/35)**

(54) **VERTICAL SOLARIUM FOR UV IRRADIATION OF A SKIN INTEGUMENT**

VERTIKALES SOLARIUM ZUR UV-BESTRAHLUNG EINES HAUTINTEGUMENTS

SOLARIUM VERTICAL D'IRRADIATION DE LA PEAU AVEC DES RAYONS U.V.

(84) Designated Contracting States:
**AT BE BG CH CY CZ DE DK EE ES FI FR GB GR HU IE IS IT LI LT LU MC NL PL PT RO SE SI SK TR**

(30) Priority: **24.02.2004 RU 2004105108**

(43) Date of publication of application:
**22.11.2006 Bulletin 2006/47**

(73) Proprietors:
• **Ryabov, Dmitry Vladislavovich**
**Moscow 125502 (RU)**
• **Mikhailov, Sergei Evgenievich**
**Moskovskaya obl., 143965 (RU)**

(72) Inventors:
• **Ryabov, Dimitry Vladislavovich**
**Moscow, 125502 (RU)**

• **Mikhailov, Sergei Evgenievich**
**Moskovskaya obl., 143965 (RU)**

(74) Representative: **Manley, Nicholas Michael**
**W.P. Thompson & Co.**
**Coopers Building**
**Church Street**
**Liverpool L1 3AB (GB)**

(56) References cited:
**EP-A1- 0 292 410      EP-A2- 1 243 288**
**WO-A1-84/04796      DE-A1- 3 317 812**
**DE-A1- 19 850 865      DE-C- 806 001**
**FR-A1- 2 371 206      SU-A- 1 084 032**
**US-A- 4 309 616**

EP 1 723 987 B1

**Description**

The present invention relates to medical engineering, more particularly, to therapeutically applicable light-irradiating devices used for treating skin diseases, such as psoriasis, Kaposi's disease, vitiligo, and others), as well as for generating vitamin $D_3$ and preventing various forms of osteoporosis. In addition, the device can find utility when used at beauty shops and sunless-tanning studios for cosmetic purposes.

One prior-art device for UV-irradiation of human's cutaneous covering, wherein the user assumes the standing posture, operating in the UV-radiation spectrum A (320-400 nm) (hereinafter referred to as "UF-A spectrum") - vertical solarium available SunVision by the firm ALISUN (both from the Netherlands) (cf. the supplement below, by the manufacturer's prospectus entitled "New vertical solarias Sun-Vision", said device comprising a body having a door and accommodating 48 fluorescent lamps for taking sunless tan, each being 2 m long and having a power output of 180 W. The lamps are spaced apart at an equal angular pitch round a common axis which is at the same time the solarium's axis, arranged parallel thereto and equidistantly therewith; in addition, the lamps have a common mirror reflector which is interposed between the lamps and the body and is spaced 10 mm apart from the lamp surface; the reflector appears as a circular cylinder having an inner mirrored surface. The device is further provided with an air-cooling system for the lamps and user. However, the device under discussion suffers from too a low efficiency and high power input.

Arrangements of UV-lamps with associated rear reflectors are disclosed in WO 84/04796 and US 4,309,616.

Taking into consideration an axial symmetry of the vertical solarium in question, user's ability to assume various positions during the session, and bearing in mind that the breadth of human's shoulder and pelvis in a majority of cases approximates 500 mm, it would be true and correct to imagine the user as a conventional convex radiation absorber appearing as a circular cylinder 500 mm in diameter and arranged coaxially with the solarium.

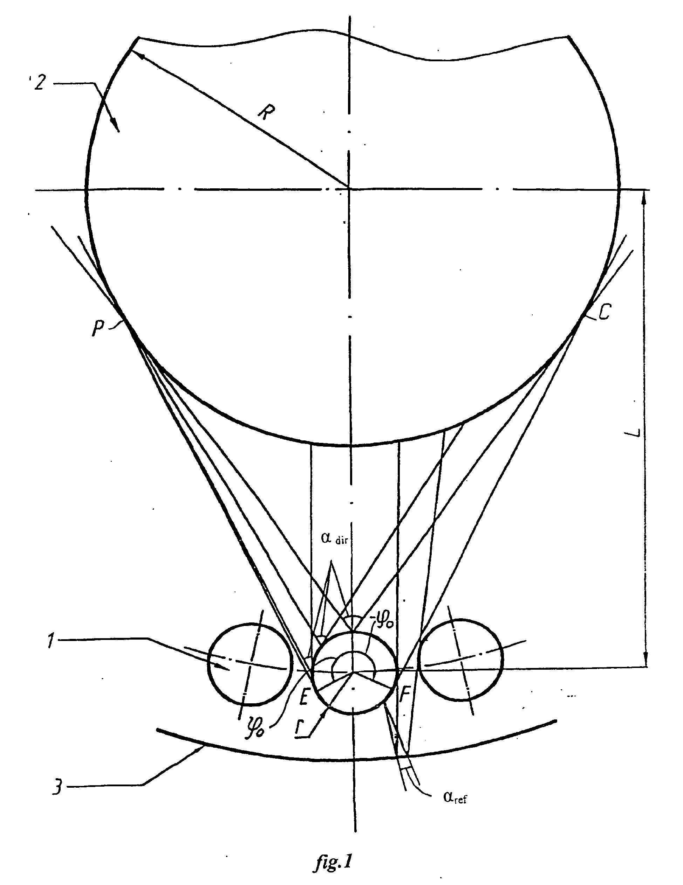

The simplifying assumptions thus made result in an axisymmetrical design model of a vertical solarium for the studying of which it is sufficient to consider a bidimensional problem (Fig.1).

Let us assume that portion of the UV radiation emitted by a lamp 1 which is incident upon an absorber 2 either directly from said lamp or after having been reflected to be a useful one, and its share in a total radiation is assumed to be an optical efficiency of the lamp (hereinafter referred to as "efficiency") which is numerically equal to the efficiency of the whole solarium on account of symmetry of the model (loss for ventilation, decorative boost lighting, and so on are left out of the given evaluation). In terms of illumination power the solarium efficiency is described by the formula:

$$(1) \quad \text{Efficiency} = 100\%(\Phi_{UVdir} + \Phi_{UVref})/\Phi_{UV},$$

wherein:

$\Phi_{UVdir}$ - direct (non-reflected) UV radiation flow incident upon the absorber;

$$\Phi_{UVdir} = \int_{\lambda_1}^{\lambda_2} \Phi_{UVdir} \, d\lambda;$$

$\Phi_{UVref}$ - reflected UV radiation flow incident upon the absorber;

$$\Phi_{UVref} = \int_{\lambda_1}^{\lambda_2} \Phi_{UVref} \, d\lambda;$$

$\Phi_{UV}$ - UV radiation flow emitted by the lamp (ratio between the energy transferred by the radiation and the transfer time exceeding considerably the oscillation period, W) :

$$\Phi_{UV} = \int_{\lambda_1}^{\lambda_2} \Phi\lambda_{UV}\, d\lambda;$$

wherein:

$\Phi\lambda_{UV}$ - spectral flow density (radiation flow per unit wavelength interval, W/nm).

Forasmuch as the lamp radiates light in every direction (i.e., diffusely) within the whole wavelength range of interest to us, equation (1) may be written in terms of angular values:

$$(2) \quad \text{Efficiency} = \frac{\alpha_{dir.av.} + \alpha_{ref.av}\, K_{ref.}}{2\pi} \cdot 100\%$$

wherein:

$\alpha_{dir.av}$ - average magnitude of angle $\alpha_{dir.}$;
$\alpha_{dir.}$ - angle at which absorber is seen from the point on the surface of the luminous element of the lamp;
$\alpha_{ref.av}$ - average magnitude of angle $\alpha_{ref.}$;
$\alpha_{ref.}$ - angle at which an airgap between lamps im reflector 3 is seen from the point on the surface of the incandescent body of the lamp;
$K_{ref.}$ - total reflection coefficient of the reflector.

It is a phosphor layer that serves as the luminous element in fluorescent lamps made use of in solaria, said layer following the geometric shape of the gas-discharge tube, and angles $\alpha_{dir.av}$ and $\alpha_{ref.av}$ are found from the following relationships:

$$(3) \quad \alpha_{dir.av} = \frac{\int_{-\varphi_o}^{\varphi_o} \alpha(\varphi)\, d\varphi}{2\varphi_o}.$$

$$(4) \quad \alpha_{dir.av} = \frac{2\int_{\varphi_1}^{\varphi_2} \alpha(\varphi)\, d\varphi}{\varphi_2 - \varphi_1}$$

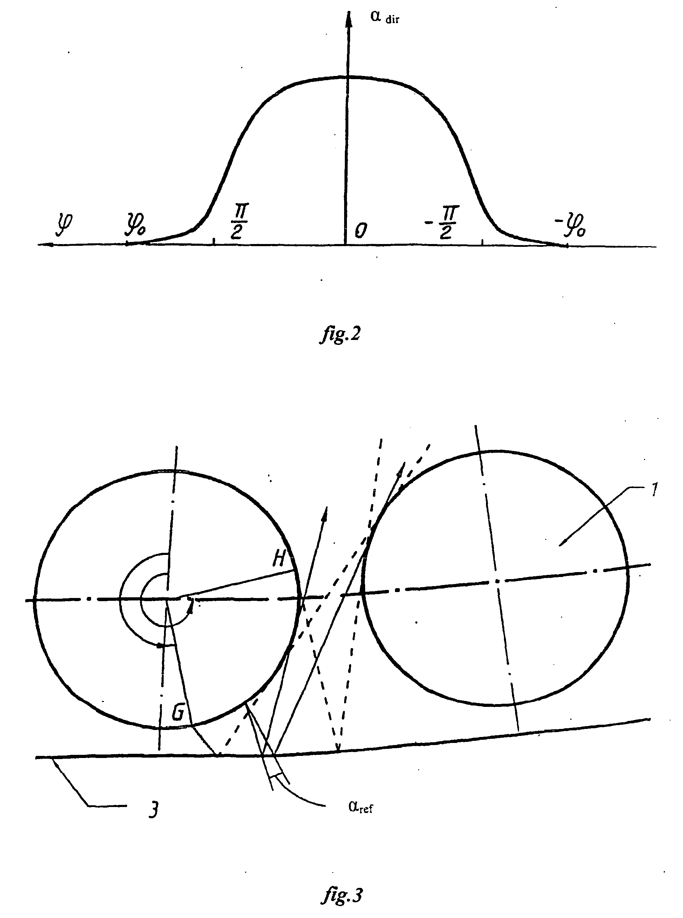

The limits of integration in formula (3) $\varphi_o$ and $-\varphi_o$ are angular coordinates of "points of sunset" E and F, i.e., such points on the lamp surface that lie on common tangents EP and CF of the lamp and absorber, respectively. All points on the lamp surface having coordinate $\varphi$ are larger than $\varphi_o$ but less than $2\pi-\varphi_o$ do not irradiate the absorber directly. The nature of dependence of angle $\alpha_{dir}$. on the angular coordinate of the radiating point on the surface of the lamp luminous element are illustrated in Fig.2.

$$(5) \qquad \varphi_o = \pi/2 + Arcsin((R-r)/L)$$

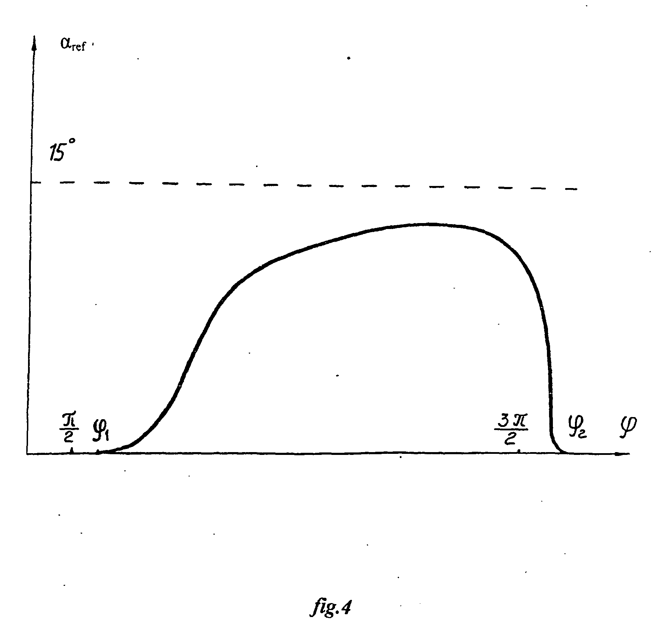

Angular coordinates of the "points of sunset" G and H for calculating $\alpha_{ref.av}$ are also determined on the basis of the solarium geometry whereby they are therein omitted. A diagram of pathways of the light rays between the lamps after their having been reflected from the reflector is shown in Fig.3. The nature of dependence of angle $\alpha_{ref}$. On the position of a point on the lamp surface is presented in Fig.4.

Having applied formulas (2), (3) and (4) for estimating efficiency of the prototype having the following dimensions: L = 440 mm, R = 250 mm, r = 20 mm, we shall obtain:

$$\alpha_{dir.av} = 70.52° \ (1.23 \ rad)$$

$$\alpha_{ref.av} = 8.95° \times 2 = 17.89° \ (0.31 \ rad)$$

$$K_{ref} = 1 \ (assuming \ the \ mirror \ to \ be \ ideal)$$

$$Efficiency = 24.59\%.$$

Herein $\Phi_{UVref}$ = 0.0497 $\Phi_{UV}$ (0.0447 $\Phi_{UV}$ for a mirror from pure aluminum having $K_{ref}$=0.9. However, reflector contribution to the efficiency of the device is but rather small, since any ray that has failed to get incident upon the absorber after first reflection will yet not be absorbed by the absorber, because the lamp-to-lamp airgaps are small and the light ray has no opportunity to have reflection twice. It is easy to verify the fact by making rather simple geometric constructions.

The results of estimations performed on the basis of a bidimensional model are corroborated by the results of measurement of irradiance in UV-A spectral range carried out by the authors on the prototype.

Hence we have ascertained that efficiency of the prototype is as low as 25% and that the reflector made use of therein directs as low as 5% of the total lamp radiation onto the absorber.

Thus, the present invention is aimed at solving a technical problem which resides in attaining higher efficiency of the device.

The technical problem of the invention is solved by reducing the amount of the lamps used and substituting the circular cylinder-shaped reflector made use of in the prototype, by a reflector comprised of 2n alternating areas of involute cylinder-shaped surfaces of two types integrated into a single surface, appearing from the evolutes of which are closed curves which limit the convex transverse sections of the absorber and lamp in contemplation.

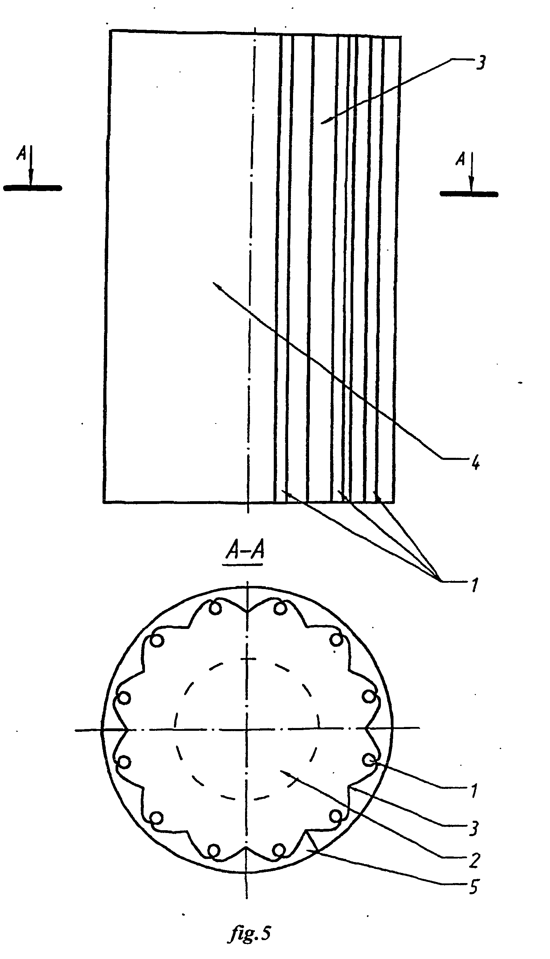

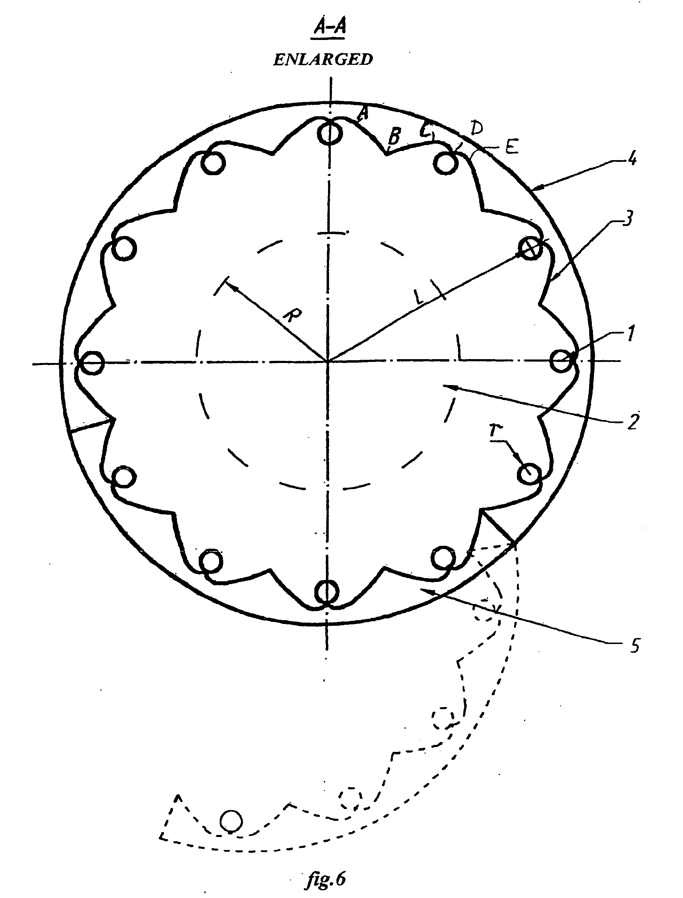

The essence of the invention is illustrated by a schematic diagram of the device with 12 lamps (Fig.5) and a luminous flux equal to that in the 48-lamp prototype. A cross-section shown in Fig.6 is conventionally enlarged.

Principal structural components of the solarium are as follows: a vertically oriented body 4 having a door 5, n fluorescent lamps for taking sunless tan, said lamps being spaced apart at an equal angular pitch round a common axis which is at the same time the solarium's axis, arranged parallel thereto and equidistantly therewith. A mirror reflector 3 is interposed between the body 4 and the lamps 1, said reflector appearing as a cylinder coaxial with the body and comprised of 2n alternating areas of involute cylinder-shaped surfaces of two types.

The areas of the first-type surface are disposed immediately behind the lamps 1 (curve CDE Fig.6) and appear as a portion of an involute cylinder-shaped surface generated by moving a straight line parallel to the solarium body axis lengthwise the straight line segments of the unlike branches of the involute of a closed curve which limits the convex transverse section of the lamp.

The areas of the second-type surface are interposed between the lamps (curve ABC in Fig.6) and appear as a portion of an involute cylinder-shaped surface generated by moving a straight line parallel to the solarium axis lengthwise the segments of the unlike branches of a convex closed curve which limits the transverse section of the conventional absorber.

The areas of the first-type and second-type surfaces are mated gently at the point B. It is due to the fact that the normal to an involute is thereto a tangent to the evolute by definition, that the herein-proposed reflector shape provides for both complete radiation emergence by preventing it from being reflected back onto the lamp, and total reflection to the absorber of all rays which have failed to get incident thereupon directly from the lamp.

The herein-proposed solarium may have as high efficiency as about 100%. It is noteworthy that efficiency of the solarium depends on the number of lamps used, since some of the luminous rays are absorbed after having got incident upon other lamps. Thus, for an ideal reflector the solarium having 6 lamps features an efficiency of 98.4%, the solarium with 12 lamps, an efficiency of 89.2%, and the solarium with 16 lamps, an efficiency of 79.7%.

Consequently, in order to get absorber illuminance in the UF-A spectrum equal to that of the prototype, it suffices 12 lamp of the same power, i.e., carrying the present invention into effect enables one to reduce electric power consumption approximately 3.6 times compared with the prototype.

## Claims

1. A vertical solarium for UV-irradiation of a human skin integument, comprising

    a circular cylinder-shaped body (4) closed along the perimeter thereof and provided with a door, said body accommodating a number n of fluorescent lamps (1) for taking sunless tan, said lamps being spaced apart at an equal angular pitch round an axis which is at the same time the solarium's axis, and being arranged parallel thereto and equidistantly therewith, said solarium further comprising a substantially cylinder-shaped mirror reflector (3) which is coaxial with the solarium body (4) and comprises parts interposed between the lamps (1) and said body,

    wherein the reflector is comprised of 2n alternating areas forming parts of first-type and second-type involute cylinder-shaped surfaces, the evolutes of said surfaces being closed curves which limit the convex cross- sections of the lamp and of a national conventional absorber (2), respectively, wherein the conventional absorber represents a user and appears as a circular cylinder of 500mm in diameter and arranged coaxially with the solarium,

    wherein each area (CDE) forming part of first-type surface is disposed immediately behind each lamp (1) and forms part of an involute cylinder-shaped surface generated by moving a straight line parallel to the axis of the solarium body (4) along the involute of a closed curve which limits the convex cross- section of the lamp (1),

    wherein each area (ABC) forming part of the second-type surface is disposed between the lamps (1) and forms part of an involute cylinder-shaped surface generated by moving a straight line parallel to the axis of the solarium body (4) along the involute of a closed curve which limits the cross section of the conventional absorber (2), and

    wherein said alternating areas are integrated to form a single surface.

## Patentansprüche

1. Vertikales Solarium für die UV-Bestrahlung eines menschlichen Hautinteguments, das Folgendes umfasst:

    einen kreiszylinderförmigen Körper (4), der entlang dem Umfang desselben geschlossen ist und mit einer Tür ausgestattet ist, wobei in dem genannten Körper eine Zahl n an Fluoreszenzlainpen (1) zum Nehmen von sonnenloser Bräune untergebracht ist, wobei die genannten Lampen in einer gleichen Winkelteilung um eine Achse beabstandet sind, die gleichzeitig die Achse des Solariums ist, und dazu parallel und in gleichen Abständen davon angeordnet sind, wobei das genannte Solarium weiter einen im Wesentlichen zylindrischen Spiegelreflektor (3) umfasst, der mit dem Solariumkörper (4) koaxial ist und zwischen den Lampen (1) und dem genannten Körper angeordnete Teile umfasst,

    wobei der Reflektor aus 2n abwechselnden Bereichen besteht, die Teile aus evolventen zylindrischen Oberflächen von erstem Typ und zweiteim Typ bilden, wobei es sich bei den Evoluten der genannten Oberflächen um geschlossene Kurven handelt, die die konvexen Querschnitte der Lampe und eines imaginären konventionellen Absorbers (2) begrenzen, wobei der konventionelle Absorber einen Benutzer darstellt und als ein Kreiszylinder mit 500 mm Durchmesser erscheint, der koaxial mit dem Solarium angeordnet ist,

    wobei jeder Bereich (CDE), der einen Teil der Oberfläche des ersten Typs bildet, direkt hinter jeder Lampe (1) angeordnet ist und einen Teil einer evolventen zylindrischen Oberfläche bildet, die erzeugt wird, in dem eine Gerade parallel zur Achse des Solariumkörpers (4) entlang der Evolute einer geschlossenen Kurve, die den konvexen Querschnitt der Lampe (1) begrenzt, verschoben wird,

    wobei jeder Bereich (ABC), der einen Teil der Oberfläche des zweiten Typs bildet, direkt zwischen den Lampen (1) angeordnet ist und einen Teil einer evolventen zylindrischen Oberfläche bildet, die erzeugt wird, in dem eine

**EP 1 723 987 B1**

Gerade parallel zur Achse des Solariumkörpers (4) entlang der Evolute einer geschlossenen Kurve, die den Querschnitt des herkömmlichen Absorbens (2) begrenzt, verschoben wird, und
wobei die genannten abwechselnden Bereiche integriert sind, um eine einzige Oberfläche zu bilden.

**Revendications**

1. Solarium vertical d'irradiation aux UV d'un tégument cutané humain, comprenant
   un circulaire de forme cylindrique (4) fermé le long de son périmètre et doté d'une porte, ledit corps recevant un nombre n de lampes fluorescentes (1) de bronzage artificiel, lesdites lampes étant espacées par un écartement angulaire égal autour d'un axe qui est en même temps l'axe du solarium, et étant agencées parallèlement et de façon équidistance avec celui-ci, ledit solarium comprenant en outre un miroir réflecteur de forme sensiblement cylindrique (3) qui est coaxial avec le corps de solarium (4) et comprend des parties interposées entre les lampes (1) et ledit corps,
   dans lequel le miroir réflecteur est composé de 2n zones alternées formant des parties de surfaces de forme cylindrique enveloppante d'un premier type et d'un second type, les développées desdites surfaces étant des courbes fermées qui limitent les coupes transversales convexes de la lampe et d'un absorbeur conventionnel notionnel (2), respectivement, l'absorbeur conventionnel représentant un utilisateur et apparaissant sous forme de cylindre circulaire de 500 mm de diamètre et agencé coaxialement avec le solarium,
   dans lequel chaque zone (CDE) formant une partie de la surface de premier type est disposée immédiatement derrière chaque lampe (1) et forme une partie d'une surface de forme cylindrique enveloppante générée en déplaçant une ligne droite parallèlement à l'axe du corps de solarium (4) le long de l'enveloppante d'une courbe fermée qui limite la coupe transversale convexe de la lampe (1),
   dans lequel chaque zone (ABC) formant une partie de la surface du second type est disposée entre les lampes (1) et forme une partie d'une surface de forme cylindrique enveloppante générée en déplaçant une ligne droite parallèlement à l'axe du corps de solarium (4) le long de l'enveloppante d'une courbe fermée qui limite la coupe transversale de l'absorbeur conventionnel (2), et
   dans lequel lesdites zones alternées sont intégrées afin de former une seule surface.

*fig.1*

fig.2

fig.3

8

fig.4

A–A

fig.5

A-A

*ENLARGED*

fig.6

**EP 1 723 987 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8404796 A

- US 4309616 A